# EUROPÄISCHE PATENTSCHRIFT

(11) **EP 2 101 666 B1**
(45) Veröffentlichungstag und Bekanntmachung des Hinweises auf die Patenterteilung: **11.07.2012**
(21) Anmeldenummer: 07856645.2
(22) Anmeldetag: 12.12.2007
(51) Int. Cl.: A61B 17/80

(54) **REPOSITIONS- UND FIXATIONSSYSTEM FÜR KNOCHENFRAGMENTE**
REPOSITIONING AND FIXATION SYSTEM FOR BONE FRAGMENTS
SYSTÈME DE REPOSITIONNEMENT ET DE FIXATION DE FRAGMENTS OSSEUX

(30) Priorität: 22.12.2006 DE 102006062164
(43) Veröffentlichungstag der Anmeldung: 23.09.2009
(73) Patentinhaber: Wolter, Dietmar, 22955 Hoisdorf (DE)
(72) Erfinder: Wolter, Dietmar, 22955 Hoisdorf (DE)
(74) Vertreter: Glawe, Delfs, Moll
(86) Internationale Anmeldenummer: PCT/EP2007/010898
(87) Internationale Veröffentlichungsnummer: WO 2008/077493

(56) Entgegenhaltungen:
- EP-A- 0 628 289
- WO-A-2004/045389
- DE-A1- 4 223 794
- DE-A1- 19 834 326
- DE-U- 7 517 440
- US-A1- 2004 159 757
- US-B1- 6 379 354

## Beschreibung

Die Erfindung betrifft ein Repositions- und Fixationssystem, mit dem Knochenfragmente reponiert und anschließend fixiert werden können.

Knochenfragmente werden seit über 100 Jahren mit Hilfe von Knochenplatten, Schrauben und Nägeln relativ zueinander fixiert. Die Fixierung von Knochenfragmenten mittels klassischer Knochenplatten setzt voraus, dass die Knochenfragmente bereits reponiert sind, das heißt in der richtigen Stellung zueinander stehen. Die klassische Knochenplatte wird auf die reponierten Knochenfragmente aufgelegt und mit Schrauben oder Nägeln an den Knochenfragmenten befestigt.

Bekannt ist weiterhin z.B. aus WO 2004/045389, welches als nächstkommender Stand der Technik angesehen wird, ein Repositions- und Fixationssystem aus zwei scharnierartig miteinander verbundenen Knochenplatten. Die beiden Knochenplatten werden mit verschiedenen Knochenfragmenten verbunden. Es ist nicht erforderlich, dass die Knochenfragmente beim Befestigen der Knochenplatten bereits in ihre endgültige Stellung reponiert sind. Vielmehr können die Knochenplatten mit den daran befestigten Knochenfragmenten um die Scharnierachse gegeneinander verschwenkt werden, um die Knochenfragmente zu reponieren. Ein solches Repositions- und Fixationssystem wird für den körperfernen Speichenbruch im Handgelenksbereich angeboten. Ein Nachteil des bekannten Repositions- und Fixationssystems besteht darin, dass es in vielen Fällen schwierig ist, die durch Verschwenken der Knochenplatten reponierten Knochenfragmente in der endgültigen Position zu fixieren. Der Erfindung liegt die Aufgabe zugrunde, ein Repositions-und Fixationssystem vorzustellen, das die Fixierung der Knochenfragmente nach dem Reponieren erleichtert. Die Aufgabe wird gelöst durch die Merkmale des Anspruchs 1. Vorteilhafte Ausführungsformen finden sich in den Unteransprüchen.

Das erfindungsgemäße Repositions- und Fixationssystem ist in Anspruch 1 definiert und umfasst mindestens zwei Knochenplatten, die dazu ausgelegt sind, miteinander verbunden zu werden. Je nach Bedarf können beliebig viele Knochenplatten miteinander verbunden werden. Jede Knochenplatte ist mit einem Ansatzelement für eine Verbindung mit einer anderen Knochenplatte ausgestattet. Das Ansatzelement einer ersten Knochenplatte umfasst eine Mehrzahl von Vorsprüngen. Das Ansatzelement einer zweiten Knochenplatte umfasst eine Öffnung. In einem ersten Verbindungszustand greifen die Vorsprünge in die Öffnung ein und stellen eine scharnierartige Verbindung zwischen der ersten Knochenplatte und der zweiten Knochenplatte her. In einem zweiten Verbindungszustand stehen die Vorsprünge unter Spannung und stellen eine starre Verbindung zwischen der ersten Knochenplatte und der zweiten Knochenplatte her.

Unter einer scharnierartigen Verbindung zweier Knochenplatten wird im Rahmen der Erfindung eine Verbindung verstanden, die als einzige Bewegung ein Verschwenken der Knochenplatten um die Scharnierachse zulässt. Bei einer starren Verbindung ist keine Bewegung der Knochenplatten relativ zueinander mehr möglich.

Mit dem erfindungsgemäßen Repositions- und Fixationssystem können an den Knochenplatten befestigte Knochenfragmente durch Verschwenken der Knochenplatten reponiert werden. Sind die Knochenfragmente in ihre endgültige Stellung gebracht, können die Vorsprünge unter Spannung gesetzt werden, so dass die Knochenplatten starr miteinander verbunden sind. Die mit den Knochenplatten verbundenen Knochenfragmente werden auf diese Weise in ihrer endgültigen Stellung fixiert.

Erfindungsgemäß weist die erste Knochenplatte eine Bohrung auf und die Vorsprünge sind am Umfang der Bohrung angeordnet. Eine solche Bohrung eröffnet die Möglichkeit, eine Schraube durch die Öffnung in der zweiten Knochenplatte und die Bohrung in der ersten Knochenplatte hindurchzuführen und mit einem angrenzenden Knochenfragment zu verschrauben. Die durch Eindrehen der Schraube erzeugte Zugkraft kann zugleich dazu genutzt werden, im zweiten Verbindungszustand die Vorsprünge unter Spannung zu setzen. Dabei greift der Kopf der Schraube an den Vorsprüngen an und setzt diese unter Spannung. Der Kopf der Schraube kann zu diesem Zweck eine kegelförmige Außenform haben.

Es ist möglich, die zum Spannen der Vorsprünge erforderliche Zugkraft durch Eindrehen der Schraube in ein Knochenfragment zu erzeugen. In vielen Fällen sind die Knochenfragmente aber spongiös und bieten dem Gewinde der Schraube keinen ausreichenden Halt. Vorteilhaft kann es deswegen sein, wenn die Schraube auch ohne Halt in einem Knochenfragment in einer starren Verbindung mit der ersten und zweiten Knochenplatte steht. Möglich ist es, dass die Öffnung in der zweiten Knochenplatte oder die Bohrung in der ersten Knochenplatte ein Gewinde aufweist, das mit dem Gewinde der Schraube zusammenwirkt. Mit Hilfe dieses Gewindes können die Vorsprünge unabhängig von der Haltekraft des angrenzenden Knochenfragments unter Spannung gesetzt werden und auf diese Weise eine starre Verbindung zwischen der ersten Knochenplatte, der zweiten Knochenplatte und der Schraube hergestellt werden.

Als vorteilhaft hat es sich erwiesen, wenn die Öffnung bzw. die Bohrung nicht bereits anfänglich mit einem Gewinde versehen ist, sondern wenn das Gewinde erst beim Eindrehen der Schraube erzeugt wird. Das Gewinde der Schraube und die Wand der Öffnung bzw. der Bohrung wirken beim Eindrehen der Schraube in der Weise zusammen, dass die Wand der Öffnung bzw. der Bohrung einer Kaltumformung unterzogen wird. Um das Eindrehen der Schraube zu erleichtern, kann die Knochenplatte in dem Bereich um die Öffnung bzw. Bohrung einen Bereich verminderter Härte aufweisen, vgl. DE 196 29 011 A1. Beispielsweise kann ein Schraubenkopf auf härterem Titanmaterial sich sich in einen aus weicherem Reintitan bestehenden Bereich der Knochenplatte einformen. Das Eindrehen der Schraube und die damit verbundene Kaltumformung kann weiter erleichtert werden, wenn die Materialstärke in diesem Bereich reduziert ist. Insbesondere kann die Wand der Öffnung bzw. Bohrung eine Verengung mit geringerer Materialstärke in Form eines Grats, einer Lippe oder einer Kante aufweisen, vgl. EP 1 211 993 B1.

Nicht zwingend erforderlich ist es, dass die Schraube senkrecht zur Knochenplatte bzw. parallel zur Bohrung eingeschraubt. Vielmehr kann die Schraube in verschiedenen Winkelstellungen in die Knochenplatte eingedreht werden, wobei dennoch eine stabile Verbindung zwischen der Knochenschraube und der Knochenplatte in dieser Winkelstellung erreicht wird, vgl. DE 198 58 889 A1, DE 43 43 117 C2. Verbindungen dieser Art werden auch als multidirektional winkelstabil bezeichnet.

Im Ausgangszustand liegen die erste Knochenplatte und die zweite Knochenplatte als getrennte Teile vor. Um die erste und die zweite Knochenplatte in den ersten Verbindungszustand zu bringen, werden die Vorsprünge der ersten Knochenplatte mit der Öffnung der zweiten Knochenplatte in Eingriff gebracht. Nachfolgend werden zwei Abschnitte der Vorsprünge unterschieden. Die Vorsprünge ragen aus der von der ersten Knochenplatte aufgespannten Ebene heraus. Ein knochenplattennaher Abschnitt eines Vorsprungs grenzt an die erste Knochenplatte an, über den knochenplattennahen Abschnitt ist der Vorsprung mit der ersten Knochenplatte verbunden. Der knochenplattenferne Abschnitt eines Vorsprungs ist von der ersten Knochenplatte abgewandt, er bildet das freie Ende eines Vorsprungs. Um einen Eingriff der Vorsprünge in die Öffnungen zu ermöglichen, können die Vorsprünge in der Weise ausgebildet sein, dass ihre knochenplattenfernen Abschnitte in einem entspannten Zustand mit einer gedachten Kreislinie verbunden werden können und dass der Durchmesser der Kreislinie größer ist als der Durchmesser Öffnung in der zweiten Knochenplatte. Beim Aufschieben der Öffnung auf die Vorsprünge werden die Vorsprünge nach innen gespannt, so dass der Durchmesser der gedachten Kreislinie sich reduziert und die Öffnung über die knochenfernen Enden der Vorsprünge hinweggeschoben werden kann.

Die knochenplattennahen Abschnitte der Vorsprünge sind vorzugsweise derart ausgebildet, dass sie in entspanntem Zustand mit einer gedachten Kreislinie verbindbar sind, deren Durchmesser dem Durchmesseer der Öffnung der zweiten Knochenplatte entspricht. Wenn die zweite Knochenplatte mit der Öffnung über die knochenplattenfernen Abschnitte hinweggeschoben wird, schnellen die Vorsprünge aus ihrer nach innen gespannten Stellung in die entspannte Stellung zurück. Die knochenplattennahen Abschnitte der Vorsprünge liegen am Innenumfang der Öffnung an. Wenn die zweite Knochenplatte gegenüber der ersten Knochenplatte geschwenkt wird, dreht sich die Innenwand der Öffnung gegenüber den Vorsprüngen. Die knochenplattennahen Abschnitte der Vorsprünge bilden zusammen mit der Innenwand der Öffnung das Lager der scharnierartigen Verbindung zwischen der ersten und der zweiten Knochenplatte.

Die Öffnung in der zweiten Knochenplatte, in die die Vorsprünge eingreifen, kann so ausgebildet sein, dass sie die Vorsprünge im ersten Verbindunggszustand rundum umgibt. Dazu kann an der Innenwand der Öffnung eine Wulst ausgebildet sein, die von den Vorsprüngen hintergriffen wird.

Die starre Verbindung, die im zweiten Verbindungszustand zwischen der ersten Knochenplatte und der zweiten Knochenplatte besteht, wird dadurch erzeugt, dass die Vorsprünge unter Spannung gesetzt werden. Die Spannung kann darin bestehen, dass die Vorsprünge gespreizt werden. Durch die Spreizung werden die Außenseiten der Vorsprünge gegen die Innenwand der Öffnung gedrückt. Die Reibung zwischen den Vorsprüngen und der Innenwand der Öffnung erhöht sich, so dass das Lager der scharnierartigen Verbindung einen erheblich erhöhten Widerstand hat. Vorteilhaft ist es, wenn die Spannung der Vorsprünge nicht nur eine nach außen gerichteten Spreizkraft bewirkt, sondern zugleich eine Kraft, durch die die zweite Knochenplatte gegen die erste Knochenplatte gedrückt wird. Eine solche kombinierte Kraftwirkung kann beispielsweise dadurch erreicht werden, dass die Vorsprünge gekippt werden. Die kombinierte Kraft führt dazu, dass sich auch die Reibung in dem Auflagebereich erhöht, in dem die erste Knochenplatte und die zweite Knochenplatte flächig aufeinanderliegen. Der widerstandserhöhende Effekt der Reibung im Auflagebereich kann weiter verstärkt werden, wenn mindestens eine der Knochenplatten im Auflagebereich Erhöhungen und Vertiefungen aufweist. Die Reibung hat den Zweck eine Schwenkbewegung zwischen der ersten Knochenplatte und der zweiten Knochenplatte zu erschweren. Die Erhöhungen und Vertiefungen setzen einer Schwenkbewegung dann den größten Widerstand entgegen, wenn sie radial zur Bohrung in der ersten Knochenplatte bzw. zur Öffnung in der zweiten Knochenplatte ausgerichtet sind.

In einer bevorzugten Ausführungsform weisen eine oder mehrere Knochenplatten mehr als ein Ansatzelement für die Verbindung mit anderen Knochenplatten auf. Dies eröffent die Möglichkeit, mehrere Knochenplatten miteinander zu verbinden. Als geeignet für die Anwendung im erfindungsgemäßen Repositions- und Fixationssystem haben sich Knochenplatten erwiesen, die eine längliche Form haben. Außer den Ansatzelementen können die Knochenplatten weitere Bohrungen aufweisen, über die Knochenplatte mit Knochenfragmenten verbunden werden kann. In diesen Bohrungen können normale Schrauben verwendet werden. Möglich ist aber auch der Einsatz von Schrauben, die sich im Wege der Kaltumformung mit den Knochenplatten verbinden, vgl. DE 198 58 889 A1, DE 43 43 117 C2. Werden einzelne Bohrungen nicht für eine Schraubverbindung benötigt, können diese frei bleibenden Bohrungen zur Stabilisierung der Knochenplatte mit Füllkörpern verschlossen werden. Dies ist aus DE 10 2004 035 546 A1 bekannt.

Nach Knochenbrüchen ist es häufig schwierig, die Knochenfragmente wieder in die richtige Stellung zueinander zu bringen. Die Knochenfragmente können durch anhaftende Muskeln stark verschoben sein. Weichteile können zwischen den Knochenfragmenten eingeklemmt sein. Der Zugang zum Bruchgebiet kann durch aufliegende Weichteile erschwert sein. In diesen Fällen kann es schwierig sein geeignete Ansatzpunkte für Repositionszangen zu finden. Bisweilen ist es erforderlich, eine Knochenplatte an ein Knochenfragment anzuschrauben, um einen Ansatzpunkt für ein Repositionswerkzeug zu schaffen.

Wenn die Knochenplatten in geeigneter Weise miteinander verbunden werden, können sie als Hebel verwendet werden, der den Vorgang des Reponierens erleichtert. Im einfachsten Fall umfasst das Repositions- und Fixationssystem zwei länglich ausgebildete Knochenplatten, die jeweils ein im Zentrum der Knochenplatte angeordnetes Ansatzelement aufweisen. Von einem im Zentrum angeordneten Ansatzelement spricht man, wenn das Ansatzelement jeweils von beiden Enden der Knochenplatte beabstandet ist. Das Gegenstück bilden an der Peripherie angeordnete Ansatzelemente. Werden die beiden Knochenplatten in ihrem Zentrum miteinander verbunden, so stellen sie ein scherenartiges Gebilde dar. Das Scharnier des scherenartigen Gebildes kann als Hebelpunkt eingesetzt werden. Zwei Enden des scherenartigen Gebildes werden dazu an zwei Knochenfragmenten befestigt. An den gegenüberliegenden Enden des scherenartigen Gebildes kann mit einer Zange angegriffen werden, um die Knochenfragmente relativ zueinander zu bewegen. Wird die Schere mit Hilfe der Zange gespreizt, so werden auch die Knochenfragmente voneinander gespreizt. Eingeklemmte Weichteile können gelöst werden. Durch Schließen der Schere können die Knochenfragmente anschließend zusammengeführt werden, so dass sie nebeneinander zu liegen kommmen. Insbesondere können die Knochenplatten auf diese Weise auch dann eingesetzt werden, wenn der Zugang zum Bruchgebiet mit großen Repositionszangen nicht möglich ist.

Alternativ kann das Repositions- und Fixationssystem auch vier Knochenplatten umfassen, die jeweils zwei an der Peripherie angeordnete Ansatzelemente aufweisen. Diese Knochenplatten können so miteinander verbunden werden, dass sie insgesamt ein Viereck bilden, dessen Schenkel scharnierartig gegeneinander verschwenkt werden können. Werden nun zwei Schenkel des Vierecks mit Knochenfragmenten verbunden, so kann mit einer Zange an den beiden anderen Knochenfragmenten angegriffen werden, um die Knochenfragmente relativ zueinander zu bewegen. Auch diese Ausführungsform des Repositions- und Fixationssystems kann vorteilhaft eingesetzt werden, wenn das Bruchgebiet für Repositionszangen nicht direkt zugänglich ist. Ferner ist diese Ausführungsform geeignet, wenn eine Vielzahl von Knochenfragmenten reponiert werden muss. Dies ist etwa bei Brüchen des Beckenknochens oder des Schulterblatts häufig der Fall.

Bei einer weiteren Ausführungsform umfasst das Repositions-und Fixationssystem wieder vier Knochenplatten, die aber jeweils ein im Zentrum und ein an der Peripherie der Knochenplatte angeordnetes Ansatzelement aufweisen. Es werden jeweils zwei Knochenplatten im Zentrum miteinander verbunden, so dass ein scherenartiges Gebilde entsteht. Die beiden Scheren werden über die peripheren Ansatzelemente zu einem scherengitterartigen Gebilde miteinander verbunden. Bei Bedarf kann eine Mehrzahl von scherenartigen Gebilden zu einem längeren Scherengitter verknüpft werden. Mit einem Repositions- und Fixationssystem in Scherengitterform kann ein größerer Abstand zwischen dem Bruchgebiet und dem Punkt, an dem die Zange angesetzt wird überbrückt werden. Möglich ist es auch, dass Scherengitter durch den Anschluss weiterer Knochengitter in der Breite zu erweitern, so dass sich flächig ausgebildetes Scherengitter ergibt. Ein flächiges Scherengitter kann insbesondere dann zum Einsatz kommen, wenn Knochenfragmente eines flächigen Knochens wie des Beckenknochens oder des Schulterblatts reponiert werden müssen.

Für eine Kraftübertragung in Längsrichtung bietet das scherengitterartige Gebilde zudem den Vorteil, dass ein Übersetzungsverhältnis geboten wird, bei dem die Zange einen großen Weg zurücklegt, während sich die mit dem gegenüberliegenden Ende verbundenen Knochenfragmente nur um eine kurze Strecke bewegen. Diese Ausführungsform ist also dann besonders geeignet, wenn eine erhebliche Kraft erforderlich ist, um die Knochenfragmente zu reponieren. Die Ausführungsformen, bei denen das Repositions- und Fixationssystem als Hebel eingesetzt wird oder einen Ansatzpunkt für eine Zange bietet, verdienen Schutz auch unabhängig von den Merkmalen des Anspruchs 1. Bei Bedarf können die scherenartigen und scherengitterartigen Gebilde auf geeignete Weise mit weiteren Knochenplatten kombiniert werden.

Die Erfindung wird nachfolgend unter Bezugnahme auf die beigefügten Zeichnungen anhand einer vorteilhaften Ausführungsform beispielhaft beschrieben. Es zeigen:
- Fig. 1:: eine erste Knochenplatte eines erfindungsgemäßen Repositions- und Fixationssystems;
- Fig. 2:: eine zweite Knochenplatte eines erfindungsgemäßen Repostionssystems;
- Fig. 3:: ein Repositions- und Fixationssystem mit den Knochenplatten aus Fig. 1 und Fig. 2 im ersten Verbindungszustand;

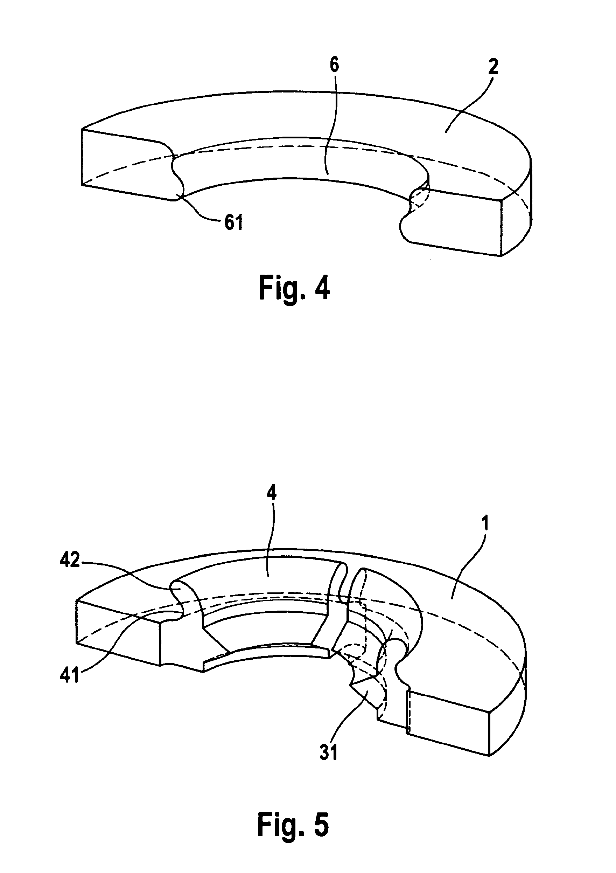

- Fig. 4:: ein Detail aus Fig. 2 in vergößerter Darstellung;
- Fig. 5:: ein Detail aus Fig. 1 in vergrößerter Darstellung;
- Fig. 6:: die Details aus Fig. 4 und Fig. 5 im ersten Verbindungszustand;
- Fig. 7:: einen Querschnitt durch ein erfindungsgemäßes Repositions- und Fixationssystem im zweiten Verbindungszustand;
- Fig. 8:: eine Draufsicht auf eine Ausführungsform einer erfindungsgemäßen Knochenplatte;
- Fig. 9:: eine Draufsicht auf eine weitere Ausführungsform einer erfindungsgemäßen Knochenplatte;
- Fig. 10:: ein Repositions- und Fixationssystem aus zwei Knochenplatten gemäß Fig. 8;
- Fig. 11:: ein Repositionsssystem bestehend aus acht Knochenplatten gemäß Fig. 9;
- Fig. 12:: ein Repositions- und Fixationssystem aus zwei Knochenplatten gemäß Fig. 8 und vier Knochenplatten gemäß Fig. 9;
- Fig. 13:: ein Repositions- und Fixationssystem aus sechs Knochenplatten gemäß Fig. 8 und vier Knochenplatten gemäß Fig. 9; und
- Fig. 14:: das Repositions- und Fixationssystem aus Fig. 13 mit zwei weiteren Knochenplatte.

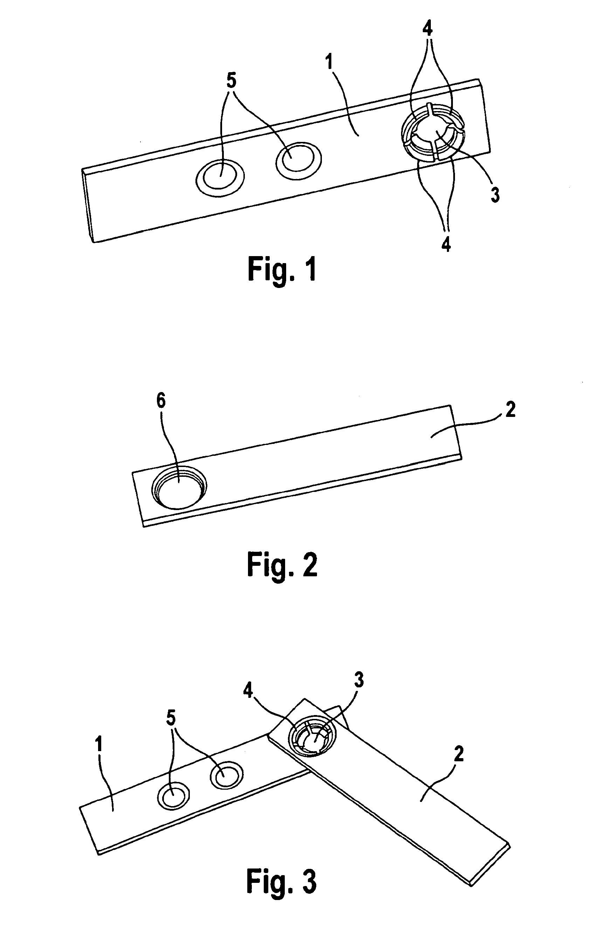

Das Repositions- und Fixationssystem umfasst eine erste Knochenplatte 1 (Fig. 1) und eine zweite Knochenplatte 2 (Fig. 2). Die erste Knochenplatte 1 weist eine Bohrung 3 und vier am Umfang der Bohrung 3 angeordnete Vorsprünge 4 auf. Die Vorsprünge 4 bilden ein Ansatzelement für eine Verbindung mit einer anderen Knochenplatte. Die Knochenplatte 1 weist weitere Bohrungen 5 auf. Die zweite Knochenplatte 2 ist mit einer Öffnung 6 ausgestattet. Die Öffnung 6 bildet ein Ansatzelement für die Verbindung mit einer weiteren Knochenplatte.

Die Vorsprünge 4 umfassen einen knochenplattennahen Abschnitt 41 und einen knochenplattenfernen Abschnitt 42. Die knochenplattenfernen Abschnitte 42 der Vorsprünge 4 können mit einer gedachten Kreislinie umgeben werden, deren Durchmesser größer ist als der kleinste Durchmesser der Öffnung 6. Der kleinste Durchmesser der Öffnung 6 wird gebildet von einer Wulst 61 in der Wand der Öffnung. Die knöchenplattennahen Abschnitte 41 der Vorsprünge 4 können mit einer gedachten Kreislinie umgeben werden, deren Durchmesser dem kleinsten Durchmesser der Öffnung 6 entspricht.

Im in den Figuren 1 und 2 gezeigten Ausgangszustand sind die Knochenplatten 1, 2 voneinander getrennt. Um die Knochenplatten in den in Fig. 3 gezeigten ersten Verbindungszustand zu bringen, werden die Vorsprünge 4 der ersten Knochenplatte 1 in die Öffnung 6 der Knochenplatte 2 eingeführt. Um die knochenplattenfernen Abschnitte 42 in die Öffnung 6 einführen zu können, müssen die Vorsprünge 4 nach innen gespannt werden, so dass sie mit einer gedachten Kreislinie umgeben werden können, deren Durchmesser nicht größer ist als der Durchmesser der Wulst 61. Nach innen gespannt werden die Vorsprünge 4, indem die Öffnung 6 auf die Vorsprünge 4 aufgesetzt wird und die erste Knochenplatte 1 und die zweite Knochenplatte 2 zusammengedrückt werden. Die Wulst 61 spannt die Vorsprünge 4 nach innen, gleitet an den knochenplattenfernen Abschnitten 42 der Vorsprünge 4 vorbei und kommt neben den knochenplattennahen Abschnitten 41 der Vorsprünge 4 zu liegen. Die Vorsprünge 4 schnellen in die entspannte Ausgangsposition zurück.

Die knochenplattenfernen Abschnitte 42 der Vorsprünge 4 hintergreifen die Wulst 61 in der Öffnung 6, so dass die zweite Knochenplatte 2 nicht ohne weiteres wieder von der ersten Knochenplatte 1 abgezogen werden kann. Die einzige Bewegung, die die zweite Knochenplatte 2 relativ zur ersten Knochenplatte 1 noch vollziehen kann, ist eine Schwenkbewegung um die Achse der Öffnung 6. Das Lager für diese Schwenkbewegung wird gebildet von der Wulst 61 und den knochenplattennahen Abschnitten 41 der Vorsprünge 4.

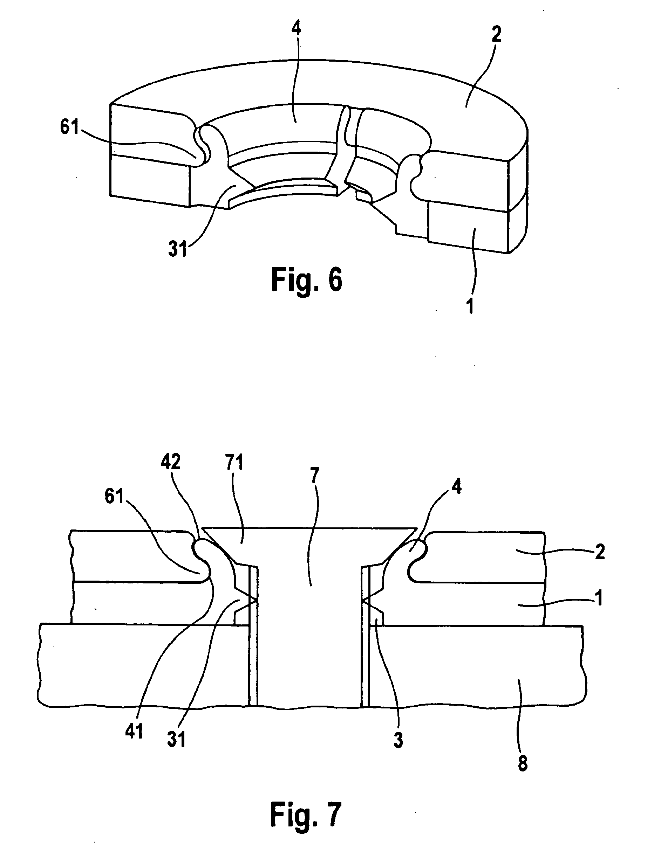

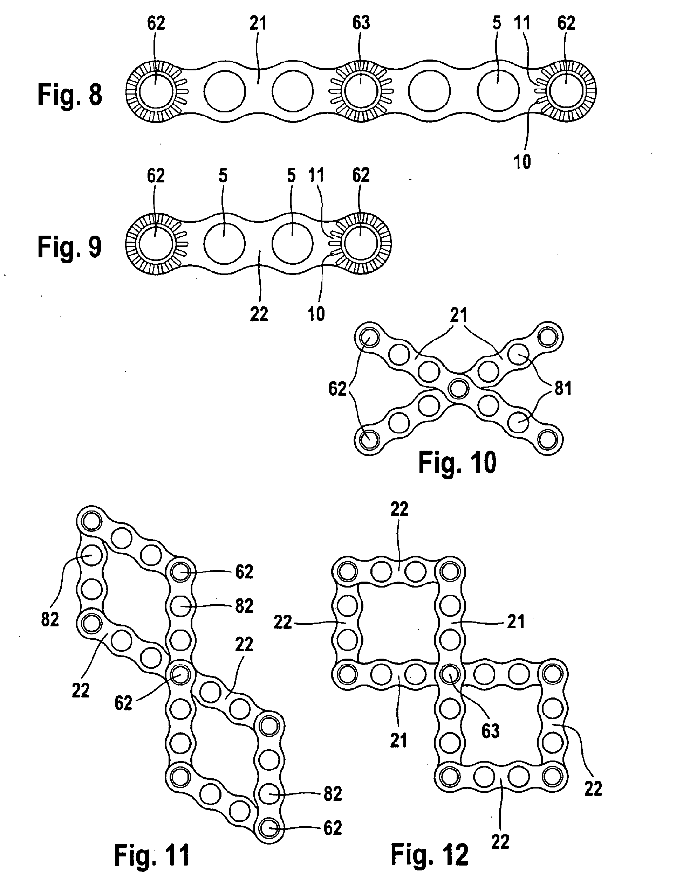

Um die Knochenplatten 1, 2 in den in Fig. 7 gezeigten zweiten Verbindungszustand zu bringen, wird eine Schraube 7 durch die Öffnung 6 in der ersten Knochenplatte 1 und die Bohrung 3 in der zweiten Knochenplatte 2 hindurchgeführt und in ein an der ersten Knochenplatte 1 anliegendes Knochenfragment 8 eingeschraubt. Durch die Zugkraft der Schraube 7 wird der Kopf 71 der Schraube 7 gegen die Vorsprünge 4 gedrückt. Die Vorsprünge 4 werden gespreizt, so dass die knochenplattennahen Abschnitte 41 der Vorsprünge 4 gegen die Wulst 61 gepresst werden. Das von der Wulst 61 und den Vorsprüngen 4 gebildete Lager wird gesperrt. Ein Verschwenken der ersten Knochenplatte 1 gegenüber der zweiten Knochenplatte 2 ist nicht mehr möglich. Zugleich bewirkt der Druck des Schraubenkopfes 71 auf die Vorsprünge 4, dass die knochenplattenfernen Enden 42 der Vorsprünge 4 umgebogen werden und von oben auf die Wulst 61 drücken. Diese Kraft bewirkt, dass die zweite Knochenplatte 2 gegen die erste Knochenplatte 1 gedrückt wird. Wie Fig. 8 zeigt, weisen die Knochenplatten 1, 2 im Auflagebereich radial von der Öffnung 6 ausgehende Erhöhungen 10 und Vertiefungen 10 auf, die die Reibung zwischen der ersten Knochenplatte 1 und der zweiten Knochenplatte 2 weiter erhöhen.

Die Bohrung 3 weist eine lippenförmige Verengung 31 auf. Der Durchmesser der von der Verengung 31 umschlossenen Öffnung ist kleiner als der Außendurchmesser der Schraube 7. Beim Eindrehen der Schraube 7 findet eine Kaltverformung der lippenförmigen Verengung 31 statt. Das Gewinde der Schraube 7 schneidet ein Gewinde in die Verengung 31, wobei sich zugleich das Material der Schraube 7 fest mit dem Material der lippenförmigen Verengung 31 verbindet. Es entsteht eine starre Verbindung zwischen der Schraube 7 und der ersten Knochenplatte 1, durch die auch die zweite Knochenplatte 2 in eine starre Verbindung mit der ersten Knochenplatte 1 gebracht wird.

Bei der Operation von Knochenbrüchen wird das beschriebene Repositions- und Fixationssystem zunächst in den ersten Verbindungszustand gebracht, Fig. 3. Durch die Bohrungen 5 kann wird die erste Knochenplatte 1 mit Schrauben an einem Knochenfragment befestigt. Es können normale Schrauben zum Einsatz kommen, die durch die Bohrung 5 hindurchgeführt werden. Es können aber auch Schrauben verwendet werden, die im Wege der Kaltumformung mit der Bohrung 5 zusammenwirken und so eine starre Verbindung mit der Knochenplatte 1 eingehen. Anschließend wird die zweite Knochenplatte 2 mit geeigneten Mitteln an einem anderen Knochenfragment befestigt, das zu diesem Zeitpunkt noch nicht in der richtigen Position relativ zum ersten Knochenfragment sein muss. Die zweite Knochenplatte 2 kann zu diesem Zweck ebenfalls Bohrungen aufweisen, die aber in Fig. 3 nicht dargestellt sind. Durch Schwenken der beiden Knochenplatten 1, 2 werden die Knochenfragmente in die passende Position zueinander gebracht. Ist die richtige Position der Knochenfragmente erreicht, werden die Vorsprünge 4 mit einer Schraube gespannt, so dass die beiden Knochenplatten relativ zueinander in starrer Position sind. Die beiden Knochenfragmente sind in ihrer Position fixiert.

Weitere Ausführungsformen der zweiten Knochenplatte sind in den Figuren 8 und 9 gezeigt. Die Knochenplatte 21 der Fig. 8 weist drei als Ansatzelemente ausgebildete Öffnungen 62, 63 sowie vier zwischen den Öffnungen 62, 63 angeordnete Bohrungen 5 auf. Die Öffnung 63 ist im Zentrum der Knochenplatte 21 angeordnet, die Öffnungen 62 sind an der Peripherie der Knochenplatte 21 angeordnet. Mit jeder der Öffnungen 62, 63 kann eine weitere Knochenplatte verbunden werden. Durch die Bohrungen 5 können Schrauben hindurchgeführt werden, die in Knochenfragmente eingeschraubt werden. Die Knochenplatte 22 der Fig. 9 weist zwei als Ansatzelemente ausgebildete Öffnungen 62 sowie zwei dazwischen liegende Bohrungen 5 auf. Zu den Knochenplatten 21, 22 analoge Knochenplatten, die anstatt der Öffnungen 62, 63 Vorsprünge 4 aufweisen, sind nicht dargestellt.

Die Knochenplatten 21, 22 können auf verschiedene Weise miteinander kombiniert werden. Einige Kombinationen sind in den Fig. 10 bis 14 gezeigt. In Fig. 10 sind zwei Knochenplatten 21 über die zentrale Öffnung 63 scherenartig miteinander verbunden. Werden die Knochenplatten 21 über die Bohrungen 81 mit Knochenfragmenten verbunden, so kann an den gegenüberliegenden Enden des scherenartigen Gebildes mit einer Zange angegriffen werden, um die Knochenfragmente zu reponieren.

In Fig. 11 sind acht Knochenplatten 22 miteinander über die peripheren Öffnungen 62 miteinander verbunden. Alle Knochenplatten 22 können relativ zueinander geschwenkt werden. Es besteht beispielsweise die Möglichkeit drei Knochenfragmente über die Bohrungen 82 festzuschrauben und dieses durch geeignetes Verschwenken der Knochenplatten 22 zu reponieren. Diese Ausführungsform ist insbesondere geeignet für flächige Knochen wie den Beckenknochen und das Schulterblatt, wo eine Mehrzahl von Knochenfragmenten zu reponieren sein kann.

Die Ausführungsform der Fig. 12 basiert auf dem in Fig. 10 gezeigten scherenartigen Gebilde. Das scherenartige Gebilde ist erweitert mit vier Knochenplatten 22. Im Unterschied zu der Ausführungsformen der Fig. 11 gibt es hier weniger Freiheitsgrade beim Verschwenken der Knochenplatten, dafür können die Knochenplatten 21 als Hebel für die Übertragung von Kräften genutzt werden.

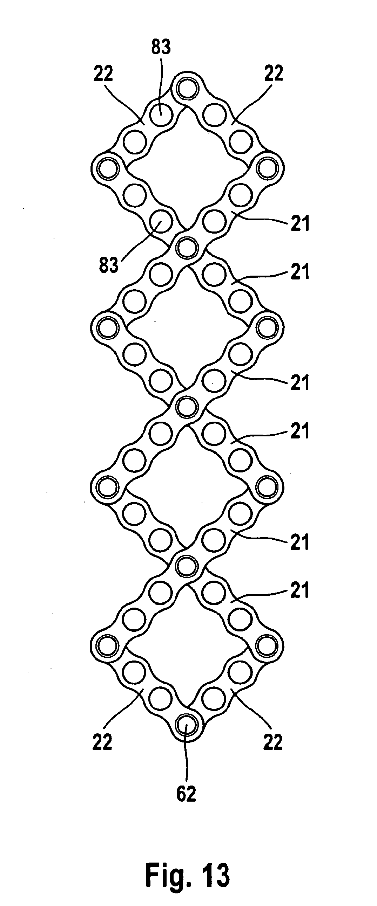

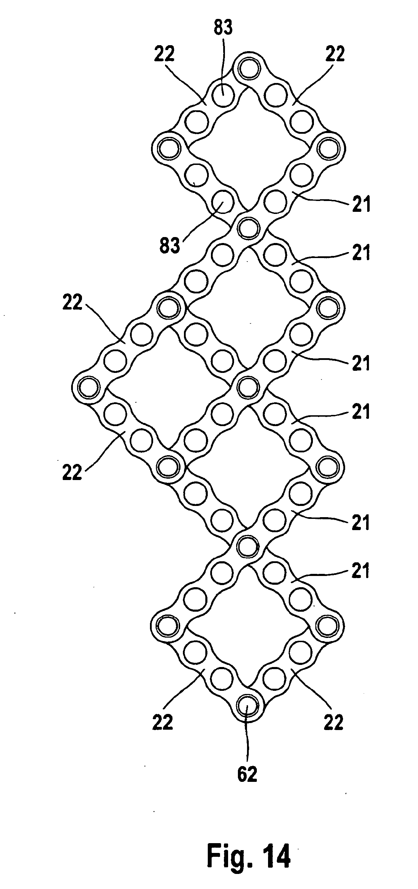

In dem Repositions- und Fixationssystem der Fig. 13 sind sechs Knochenplatten 21 mit zwei Knochenplatten 22 zu einem scherengitterartigen Gebilde verbunden. Das Scherengitter bietet eine Übersetzungsverhältnis für die Übertragung von Längskräften. Werden etwa zwei Knochenfragmente mit den Bohrungen 83 verbunden und wird mit einer Zange am gegenüberliegenden Ende des Scherengitters gezogen, so ist die auf die Knochenfragmente wirkende Zugkraft wesentlich größer als die von der Zange ausgeübte Kraft. In Fig. 14 ist das Scherengitter aus Fig. 13 mit zwei Knochenplatten 22 erweitert.

## Patentansprüche

1. Repositions- und Fixationssystem für Knochenfragmente, umfassend zwei miteinander verbindbare Knochenplatten (1, 2), wobei jede Knochenplatte (1, 2) ein Ansatzelement (4, 6) für eine Verbindung mit einer anderen Knochenplatte (1, 2) aufweist, mit folgenden Merkmalen:
a) das Ansatzelement einer ersten Knochenplatte (1) umfasst eine Bohrung (3);
b) das Ansatzelement einer zweiten Knochenplatte (2) umfasst eine Öffnung (6); **gekennzeichnet durch** die weiteren Merkmale:
c) das Ansatzelement der ersten Knochenplatte (1) umfasst ferner eine Mehrzahl von Vorsprüngen (4), die am Umfang der Bohrung (3) angeordnet sind;
d) in einem ersten Verbindungszustand greifen die Vorsprünge (4) in die Öffnung (6) ein und stellen eine scharnierartige Verbindung zwischen der ersten Knochenplatte (1) und der zweiten Knochenplatte (2) her;
e) in einem zweiten Verbindungszustand stehen die Vorsprünge (4) unter Spannung und stellen eine starre Verbindung zwischen der ersten Knochenplatte (1) und der zweiten Knochenplatte (2) her; und
f) es ist eine Schraube (7) vorgesehen, die im zweiten Verbindungszustand **durch** die Öffnung (6) in der zweiten Knochenplatte (2) und **durch** die Bohrung (3) in der ersten Knochenplatte (1) hindurchgeführt ist, so dass der Kopf (71) der Schraube (7) die Vorsprünge (4) unter Spannung setzt

2. Repositions- und Fixationssystem nach Anspruch 1, **dadurch gekennzeichnet, dass** die Schraube (7) im zweiten Verbindungszustand in einer starren Verbindung mit der ersten Knochenplatte (1) und der zweiten Knochenplatte (2) steht.

3. Repositions- und Fixationssystem nach Anspruch 2, **dadurch gekennzeichnet, dass** die starre Verbindung auf einer beim Eindrehen der Schraube (7) bewirkten Kaltumformung beruht.

4. Repositions- und Fixationssystem nach einem der Ansprüche 1 bis 3, **dadurch gekennzeichnet, dass** die Vorsprünge (4) knochenplattenfernen Abschnitte (42) aufweisen, dass die knochenplattenfernen Abschnitte (42) der Vorsprünge (4) in einem entspannten Zustand mit einer gedachten Kreislinie verbindbar sind und dass der Durchmesser der Kreislinie größer ist als der Durchmesser der Öffnung (6) der zweiten Knochenplatte (2).

5. Repositions- und Fixationssystem nach einem der Ansprüche 1 bis 4, **dadurch gekennzeichnet, dass** die Vorsprünge (4) knochenplattennahe Abschnitte (41) aufweisen, dass die knochenplattennahen Abschnitte (41) der Vorsprünge (4) in einem entspannten Zustand mit einer gedachten Kreislinie verbindbar sind und dass der Durchmesser der Kreislinie dem Durchmesser der Öffnung (6) der zweiten Knochenplatte (2) entspricht.

6. Repositions- und Fixationssystem nach einem der Ansprüche 1 bis 5, **dadurch gekennzeichnet, dass** die erste Knochenplatte (1) und die zweite Knochenplatte (2) im zweiten Verbindungszustand in einem Auflagebereich flächig aufeinanderliegen und dass mindestens eine der Knochenplatten (1, 2) im Auflagebereich Erhöhungen (10) und Vertiefungen (11) aufweist.

7. Repositonssystem nach Anspruch 6, **dadurch gekennzeichnet, dass** die Erhöhungen (10) und Vertiefungen (11) radial zur Bohrung (3) in der ersten Knochenplatte (1) bzw. zur Öffnung (6) in der zweiten Knochenplatte (2) ausgerichtet sind.

8. Repositions- und Fixationssystem nach einem der Ansprüche 1 bis 7, **dadurch gekennzeichnet, dass** die Knochenplatte (21, 22) mehr als ein Ansatzelement (62, 63) aufweist.

9. Repositions- und Fixationssystem nach einem der Ansprüche 1 bis 8, **dadurch gekennzeichnet, dass** die Knochenplatte (21, 22) eine längliche Form hat.

10. Repositions- und Fixationssystem nach Anspruch 9, **dadurch gekennzeichnet, dass** die Knochenplatten (21) ein im Zentrum der Knochenplatte (21) angeordnetes Ansatzelement (63) aufweisen.

11. Repositions- und Fixationssystem nach Anspruch 9, **dadurch gekennzeichnet, dass** es vier Knochenplatten (22) umfasst und dass jede Knochenplatte (22) zwei an der Peripherie der Knochenplatte angeordnete Ansatzelemente (62) umfasst.

12. Repositions- und Fixationssystem nach Anspruch 9, **dadurch gekennzeichnet, dass** es vier Knochenplatten (21) umfasst und dass jede Knochenplatte (21) ein im Zentrum der Knochenplatte angeordnetes Ansatzelement (63) und ein an der Peripherie der Knochenplatte angeordnetes Ansatzelement (62) umfasst.

## Claims

1. Repositioning and fixation system for bone fragments, comprising two interconnectable bone plates (1, 2), each bone plate (1, 2) having a connector element (4, 6) for connection to another bone plate (1, 2), with the following features:
a) the connector element of a first bone plate (1) comprises a bore (3);
b) the connector element of a second bone plate (2) comprises an opening (6);
**characterized by** the further features:
c) the connector element of the first bone plate (1) further comprises a plurality of projections (4), which are arranged on the circumference of the bore (3) ;
d) in a first connection state, the projections (4) engage in the opening (6) and produce a hinge-type connection between the first bone plate (1) and the second bone plate (2);
e) in a second connection state, the projections (4) are tensioned and produce a rigid connection between the first bone plate (1) and the second bone plate (2); and
f) a screw (7) is provided, which, in the second connection state, is guided through the opening (6) in the second bone plate (2) and through the bore (3) in the first bone plate (1), such that the head (71) of the screw (7) subjects the projections (4) to tensioning.

2. Repositioning and fixation system according to Claim 1, **characterized in that** the screw (7), in the second connection state, is in rigid connection to the first bone plate (1) and to the second bone plate (2).

3. Repositioning and fixation system according to Claim 2, **characterized in that** the rigid connection is based on a cold-forming that takes place as the screw (7) is screwed in.

4. Repositioning and fixation system according to one of Claims 1 through 3, **characterized in that** the projections (4) have segments (42) remote from the bone plate, **in that** the segments (42) of the projections (4) remote from the bone plate can be connected, in an untensioned state, to an imaginary circle, and **in that** the diameter of the circle is greater than the diameter of the opening (6) of the second bone plate (2).

5. Repositioning and fixation system according to one of Claims 1 through 4, **characterized in that** the projections (4) have segments (41) close to the bone plate, **in that** the segments (41) of the projections (4) close to the bone plate can be connected, in an untensioned state, to an imaginary circle, and **in that** the diameter of the circle corresponds to the diameter of the opening (6) of the second bone plate (2).

6. Repositioning and fixation system according to one of Claims 1 through 5, **characterized in that** the first bone plate (1) and the second bone plate (2), in the second connection state, lie flat on each other in a contact area, and **in that** at least one of the bone plates (1, 2) has elevations (10) and depressions (11) in the contact area.

7. Repositioning and fixation system according to Claim 6, **characterized in that** the elevations (10) and depressions (11) are oriented radially with respect to the bore (3) in the first bone plate (1) and/or to the opening (6) in the second bone plate (2).

8. Repositioning and fixation system according to one of Claims 1 through 7, **characterized in that** the bone plate (21, 22) has more than one connector element (62, 63).

9. Repositioning and fixation system according to one of Claims 1 through 8, **characterized in that** the bone plate (21, 22) has an elongate shape.

10. Repositioning and fixation system according to Claim 9, **characterized in that** the bone plates (21) have a connector element (63) arranged at the center of the bone plate (21).

11. Repositioning and fixation system according to Claim 9, **characterized in that** it comprises four bone plates (22), and **in that** each bone plate (22) comprises two connector elements (62) arranged at the periphery of the bone plate.

12. Repositioning and fixation system according to Claim 9, **characterized in that** it comprises four bone plates (21), and **in that** each bone plate (21) comprises a connector element (63) arranged at the center of the bone plate and a connector element (62) arranged at the periphery of the bone plate.

## Revendications

1. Système de repositionnement et de fixation pour des fragments osseux, comprenant deux plaques osseuses (1, 2) pouvant être reliées l'une à l'autre, chaque plaque osseuse (1, 2) présentant un élément rapporté (4, 6) pour une liaison avec une autre plaque osseuse (1, 2), présentant les caractéristiques suivantes :
a) l'élément rapporté d'une première plaque osseuse (1) comprend un perçage (3) ;
b) l'élément rapporté d'une seconde plaque osseuse (2) comprend une ouverture (6) ;
**caractérisé par** les autres caractéristiques suivantes :
c) l'élément rapporté de la première plaque osseuse (1) comprend également une pluralité de saillies (4), qui sont disposées sur le pourtour du perçage (3) ;
d) dans un premier état de liaison, les saillies (4) s'engagent dans l'ouverture (6) et établissent une liaison de type charnière entre la première plaque osseuse (1) et la seconde plaque osseuse (2) ;
e) dans un second état de liaison, les saillies (4) sont sous tension et établissent une liaison solide entre la première plaque osseuse (1) et la seconde plaque osseuse (2) ; et
f) il est prévu une vis (7), qui est guidée, dans le second état de liaison, à travers l'ouverture (6) dans la seconde plaque osseuse (2) et à travers le perçage (3) dans la première plaque osseuse (1), de sorte que le sommet (71) de la vis (7) met les saillies (4) sous tension.

2. Système de repositionnement et de fixation selon la revendication 1, **caractérisé en ce que**, dans le second état de liaison, la vis (7) est dans une liaison fixe avec la première plaque osseuse (1) et la seconde plaque osseuse (2).

3. Système de repositionnement et de fixation selon la revendication 2, **caractérisé en ce que** la liaison fixe repose sur un formage à froid provoqué lors du vissage de la vis (7).

4. Système de repositionnement et de fixation selon l'une des revendications 1 à 3, **caractérisé en ce que** les saillies (4) présentent des parties (42) éloignées de la plaque osseuse, **en ce que** les parties (42), éloignées de la plaque osseuse, des saillies (4) peuvent être reliées dans un état détendu à une ligne de cercle imaginaire et **en ce que** le diamètre de la ligne de cercle est supérieur au diamètre de l'ouverture (6) de la seconde plaque osseuse (2).

5. Système de repositionnement et de fixation selon l'une des revendications 1 à 4, **caractérisé en ce que** les saillies (4) présentent des parties (41) proches de la plaque osseuse, **en ce que** les parties (41), proches de la plaque osseuse, des saillies (4) peuvent être reliées dans un état détendu avec une ligne de cercle imaginaire et **en ce que** le diamètre de la ligne de cercle correspond au diamètre de l'ouverture (6) de la seconde plaque osseuse (2).

6. Système de repositionnement et de fixation selon l'une des revendications 1 à 5, **caractérisé en ce que** la première plaque osseuse (1) et la seconde plaque osseuse (2) se situent l'une au-dessus de l'autre à plat dans une zone d'appui dans le second état de liaison et **en ce qu'**au moins l'une des plaques osseuses (1, 2) présente dans la zone de support des élévations (10) et des cavités (11).

7. Système de repositionnement selon la revendication 6, **caractérisé en ce que** les élévations (10) et les cavités (11) sont orientées radialement par rapport au perçage (3) dans la première plaque osseuse (1) et par rapport à l'ouverture (6) dans la seconde plaque osseuse (2).

8. Système de repositionnement et de fixation selon l'une des revendications 1 à 7, **caractérisé en ce que** la plaque osseuse (21, 22) présente plus d'un élément rapporté (62, 63).

9. Système de repositionnement et de fixation selon l'une des revendications 1 à 8, **caractérisé en ce que** la plaque osseuse (21, 22) a une forme allongée.

10. Système de repositionnement et de fixation selon la revendication 9, **caractérisé en ce que** les plaques osseuses (21) présentent un élément rapporté (63) disposé au centre de la plaque osseuse (21).

11. Système de repositionnement et de fixation selon la revendication 9, **caractérisé en ce qu'**il comprend quatre plaques osseuses (22) et **en ce que** chaque plaque osseuse (22) présente deux éléments rapportés (62) disposés sur la périphérie de la plaque osseuse.

12. Système de repositionnement et de fixation selon la revendication 9, **caractérisé en ce qu'**il comprend quatre plaques osseuses (21) et **en ce que** chaque plaque osseuse (21) comprend un élément rapporté (63) disposé au centre de la plaque osseuse et comprend un élément rapporté (62) disposé à la périphérie de la plaque osseuse.
